# EUROPEAN PATENT SPECIFICATION

(11) **EP 2 019 092 B1**
(45) Date of publication and mention of the grant of the patent: **03.07.2013**
(21) Application number: 07743661.6
(22) Date of filing: 18.05.2007
(51) Int. Cl.: C07C 209/68, C07C 211/49

(54) **PROCESS FOR PRODUCING POLYAMINE**
VERFAHREN ZUR HERSTELLUNG VON POLYAMIN
PROCÉDÉ DE PRODUCTION DE POLYAMINE

(30) Priority: 19.05.2006 JP 2006139649
(43) Date of publication of application: 28.01.2009
(73) Proprietor: Mitsui Chemicals, Inc., Tokyo (JP)
(72) Inventor: OHKUBO, Tsuneyuki, Sodegaura-shi, Chiba 299-0265 (JP); OHTANI, Rieko, Takaishi-shi, Osaka 592-8501 (JP)
(74) Representative: HOFFMANN EITLE
(86) International application number: PCT/JP2007/060226
(87) International publication number: WO 2007/135988

(56) References cited:
- JP-A- 2003 313 155
- JP-A- 2004 359 672
- JP-A- 2006 083 103

## Description

### TECHNICAL FIELD

The present invention relates to a process for producing a polyamine, and more specifically, to a process for producing a polyamine such as diaminodiphenylmethane and polymethylene polyphenylene polyamine.

### BACKGROUND ART

As conventional methods for producing a polyamine such as diaminodiphenylmethane and polymethylene polyphenylene polyamine, a method for rearranging N,N'-diphenylmethylenediamine, which is a condensate of formaldehyde and aniline, is known.
There has been proposed, for example, a process in which N,N'-diphenylmethylenediamine is rearranged by heating in the presence of an organic sulfonic acid group-containing polysiloxane, which is a solid acid catalyst, to give a reaction liquid containing diaminodiphenylmethane and polymethylene polyphenylene polyamine, the reaction liquid is then cooled, the polysiloxane is filtered off and recovered, and the recovered polysiloxane is used again (cf. for example the following patent document 1).
Patent Document 1: Japanese Unexamined Patent Publication No. 2006-83103

### DISCLOSURE OF THE INVENTION

### Problems to be Solved

However, the process described in the patent document 1 has a problem in that, in the case of repeatedly using polysiloxane, although deterioration of catalytic activity is prevented to some extent, the prevention of such deterioration is not yet industrially satisfactory.
It is an object of the present invention to provide a process for producing a polyamine such as diaminodiphenylmethane and polymethylene polyphenylene polyamine, capable of reliably preventing deterioration of catalytic activity and decline in selectivity, even though polysiloxane is repeatedly used.

### MEANS FOR SOLVING THE PROBLEM

To achieve the above objects, the process for producing a polyamine comprises a rearrangement reaction step of:
subjecting a condensate of aniline and formaldehyde to a rearrangement reaction in the presence of an organic sulfonic acid group-containing polysiloxane to form a product containing diaminodiphenylmethane, polymethylene polyphenylene polyamine and a by-product;
a product withdrawal step of partially withdrawing the product solution in an amount 1 to 90 parts by weight per 100 parts by weight of a product solution; and
a reduction step of reducing a peak area of the by-product to 1.0% or less relative to a total peak area of a peak area of the by-product, a peak area of the diaminodiphenylmethane, a peak area of the polymethylene polyphenylene polyamine and a peak area of the aniline when the product is measured by liquid chromatography using a UV detector at a wavelength of 200 nm
wherein the reduction is carried out by heating the product solution containing the polysiloxane remaining after the product withdrawal step.

Further, in the polyamine producing process according to the present invention, it is preferable that the condensate is dissolved in aniline in the rearrangement reaction step.

Further, in the polyamine producing process according to the present invention, it is preferable that the polysiloxane is formed in the form of a particle having pores, and a pore having a pore size of 2 to 50 nm is 20% by volume or less per a pore having a pore size of 0.9 to 50 nm.
Further, in the polyamine producing process according to the present invention, it is preferable that the diaminodiphenylmethane is 4,4'-diaminodiphenylmethane and/or 2,4'-diaminodiphenylmethane; the polymethylene polyphenylene polyamine is a compound represented by the following formula(s) (1) and/or (2); and the by-product is at least one selected from N-(4-aminobenzyl)aniline, N-(2-aminobenzyl)aniline, N-methyl-4,4'-methylenedianiline and compounds represented by the following formulas (3) to (5):

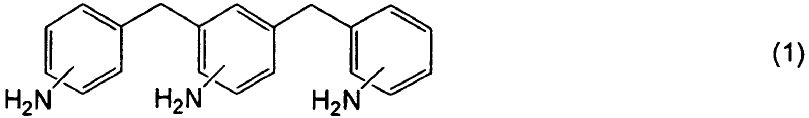

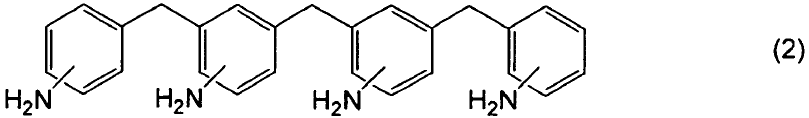

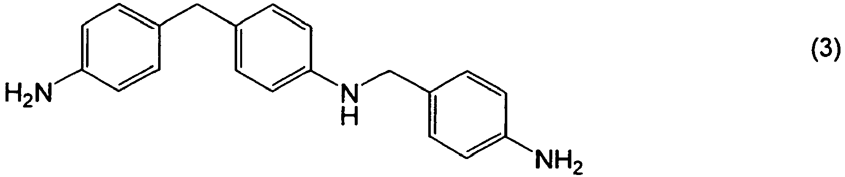

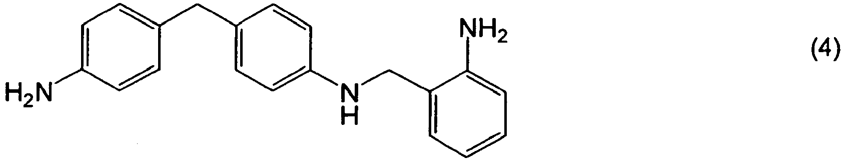

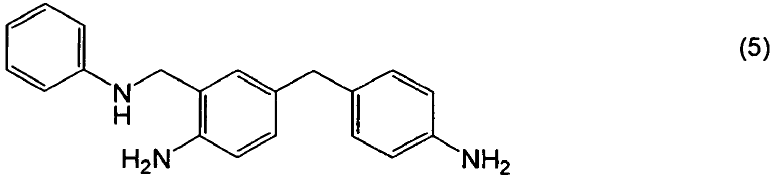

### EFFECT OF THE INVENTION

According to the polyamine production process of the present invention, even though polysiloxane is repeatedly used, deterioration of catalytic activity and decline in selectivity can be reliably prevented. Therefore, polyamine such as diaminodiphenylmethane and polymethylene polyphenylene polyamine can be produced with a high yield and excellent selectivity over a long period of time.

### BRIEF DESCRIPTION OF THE DRAWINGS

[FIG.1] FIG. 1 is a flowchart illustrating one embodiment of a polyamine production process according to the present invention;
[FIG.2] FIG. 2 shows a polyamine producing apparatus for specifically performing the polyamine production process in FIG. 1;
[FIG.3] FIG. 3 shows a polyamine producing apparatus equipped with a filtering device, for specifically performing the polyamine production process in FIG. 1; and
[FIG.4] FIG. 4 shows a polyamine producing apparatus equipped with a catalytic reaction bath, for specifically performing the polyamine production process in FIG. 1.

### EMBODIMENT OF THE INVENTION

The polyamine production process of the present invention includes a rearrangement reaction step of subjecting a condensate of aniline and formaldehyde to a rearrangement reaction in the presence of polysiloxane to form a product containing a polyamine as a main product and a by-product; and a by-product reduction step of reducing a peak area of the by-product when the product is measured by liquid chromatography.
The condensate of aniline and formaldehyde has a molar ratio of aniline to formaldehyde of, for example, 2:1, and primarily contains N,N'-diphenylmethylenediamine. More specifically, such condensate contains N,N'-diphenylmethylenediamine as a main reaction component in the condensation reaction, and aniline or/and formaldehyde as an unreacted component(s) thereof.

The condensate is obtained by mixing aniline with an aqueous solution of formaldehyde (formalin) so that aniline is contained in an amount of 2 parts by mol or more, or preferably 3 to 6 parts by mol or more, per 1 part by mol of formaldehyde, and condensing these components to give a two-phase mixture made of an organic layer and an aqueous layer. Thereafter, the organic layer and the aqueous layer are separated (liquid separation), and the separated organic layer is purified.
The condensate after the purification contains water in an amount of, for example, 3 parts by weight or less, or preferably 1. 5 parts by weight or less, per 100 parts by weight of the condensate. The amount of N,N'-diphenylmethylenediamine contained in the condensate is, for example, from 25 to 100 parts by weight, or preferably from 30 to 70 parts by weight, per 100 parts by weight of the condensate.

The condensate can be produced according to known methods, for example, the method described in the paragraph of [0006] in Japanese Unexamined Patent Publication No. 2006-83103.
The polysiloxane has an organic sulfonic acid group, and is a catalyst, more specifically, a solid acid catalyst, used for the rearrangement reaction of the condensate. The polysiloxane contains a siloxane skeleton (-[SiO]ₙ-) as a main chain and an organic sulfonic acid group as a side chain bonded to a silicon atom in the main chain.

The organic sulfonic acid group is, for example, a hydrocarbon group having a sulfonic acid group (-SO₃H), and one end of the hydrocarbon group is bonded to a silicon atom in the main chain, and the other end thereof is bonded to a sulfonic acid group. The hydrocarbon group has, for example, 6 to 20 carbon atoms, and examples thereof include aliphatic hydrocarbon group, alicyclic hydrocarbon group and aromatic hydrocarbon group. An aromatic hydrocarbon group such as a phenyl group is preferable. The hydrocarbon group may have a substituent (substituent except sulfonic acid group), and examples of the substituent include a halogen atom, an alkoxy group, a nitro group and a hydroxy group. The number of sulfonic acid groups contained in one hydrocarbon group is, for example, one or more, and is usually two or less. Therefore, the content ratio of the sulfonic acid group in the polysiloxane is set in the range of, for example, 0.01 to 3.0 meq/g.

The polysiloxane is, for example, in the form of a particle, or preferably in the form of a particle having pores. More specifically, such polysiloxane is formed from secondary particles in which primary particles are randomly joined to one another. Examples of the pore in the polysiloxane include micropores formed in the surface (outermost surface layer) of the primary particle and voids formed between the primary particles.

The pores in the polysiloxane include pores having a pore size of 2 to 50 nm in an amount of, for example, 20% by volume or less, preferably 10% by volume or less, or more preferably 5% by volume or less, to pores having a pore size of 0.9 to 50 nm. Even more preferably, the polysiloxane is formed so that the pores having a pore size of 2 to 50 nm are substantially 0% by volume to the pores having a pore size of 0.9 to 50 nm, that is, the pores having a pore size of 2 to 50 nm are absent.

The pores having a pore size of 0.9 to 50 nm are formed of micropores in the surface of the primary particle and voids formed between the primary particles, and the pores having a pore size of 2 to 50 nm are formed of voids formed between the primary particles. Generally, the micropore in the surface of the primary particle is a micro-scale pore, and the void between the primary particles is a meso-scale pore. In other words, the pores in the polysiloxane include the meso-scale pore in an amount of, for example, 20% by volume or less, preferably 10% by volume or less, or more preferably 5% by volume or less, of the total volume of pore including micro-scale pores and meso-scale pores. Even more preferably, polysiloxane is formed so that meso-scale pores are substantially absent, that is, the surface of the polysiloxane has non-meso-scale pore formed.

When the volume ratio of the pore (meso-scale pore) having a pore size of 2 to 50 nm is within the above range, deterioration of catalytic activity due to clogging of pores with the product during the repeated use of polysiloxane can be prevented, that is, deterioration of catalytic activity can be suppressed by improvement in the diffusibility of the product on the surface of polysiloxane.
The specific surface area of the polysiloxane having the above-mentioned pore is set in the range of, for example, 400 to 1500 m²/g.

In polysiloxane, the volume and the specific surface area of the pore having a pore size of 0.9 to 50 nm are calculated from the measurement results of the pore distribution by nitrogen adsorption method.
The polysiloxane is produced, for example, by a method of preliminarily synthesizing a hydrocarbon group-containing polysiloxane followed by sulfonation thereof, or a method of hydrolyzing an organic sulfonic acid group-containing alkoxysilane. Preferably, the polysiloxane is produced by the method of hydrolyzing an organic sulfonic acid group-containing alkoxysilane. The polysiloxane can be produced according to known methods, for example, the method described in the paragraphs of [0007] to [0011] in Japanese Unexamined Patent Publication No. 2006-83103.

In the present invention, examples of the polyamine as the main product include diaminodiphenylmethane and polymethylene polyphenylene polyamine.
Examples of the diaminodiphenylmethane include 2,2'-diaminodiphenylmethane, 2,3'-diaminodiphenylmethane, 2,4'-diaminodiphenylmethane, 3,3'-diaminodiphenylmethane, 3,4'-diaminodiphenylmethane and 4,4'-diaminodiphenylmethane. From the viewpoint of orientation, 2,4'-diaminodiphenylmethane and 4,4'-diaminodiphenylmethane are preferable. The diaminodiphenylmethane may be made of two kinds of the above-mentioned compounds.

Examples of the polymethylene polyphenylene polyamine include compounds having a plurality of aniline moieties represented by the following general formula (6):

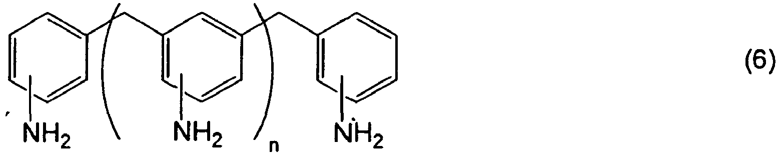

(wherein n represents an integer of one or more.)

Preferable examples of the polymethylene polyphenylene polyamine include compounds represented by the following formula (1) or (2):

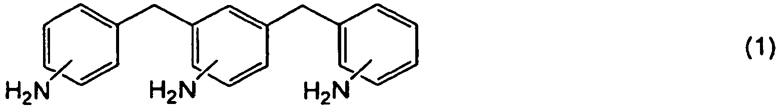

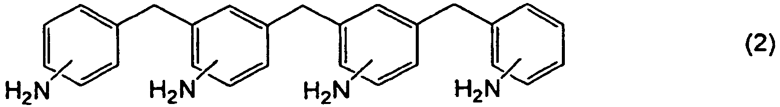

The polymethylene polyphenylene polyamine may be made of two kinds of the above-mentioned compounds.
In the present invention, the by-product is, for example, a compound having an aniline moiety, which is produced at the mid-way of a rearrangement reaction from a condensate to a main product, and more specific examples thereof include N-(4-aminobenzyl)aniline, N-(2-aminobenzyl)aniline, N-methyl-4,4'-methylenedianiline and compounds represented by the following formulas (3) to (5):

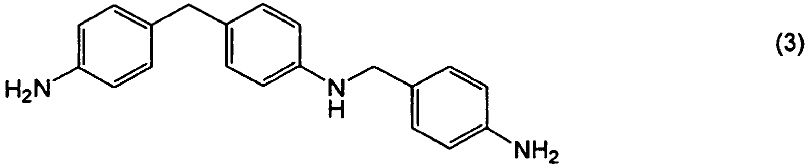

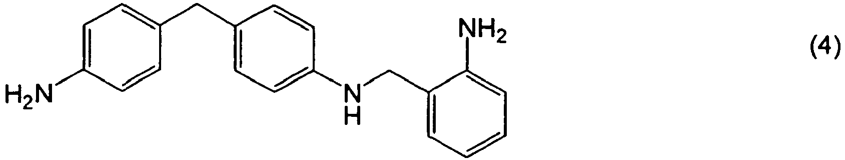

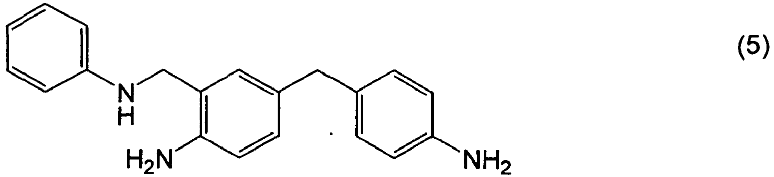

The by-product may be made of two or more kinds of the above-mentioned compounds.
The aniline moiety which each of the above-mentioned compounds has includes an aminophenyl group represented by the following formula (7) ; an anilino group represented by the following formula (8) and an N-methyl-aminophenyl group represented by the following formula (9).

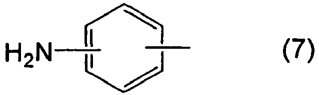

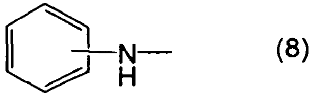

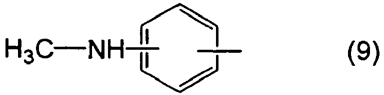

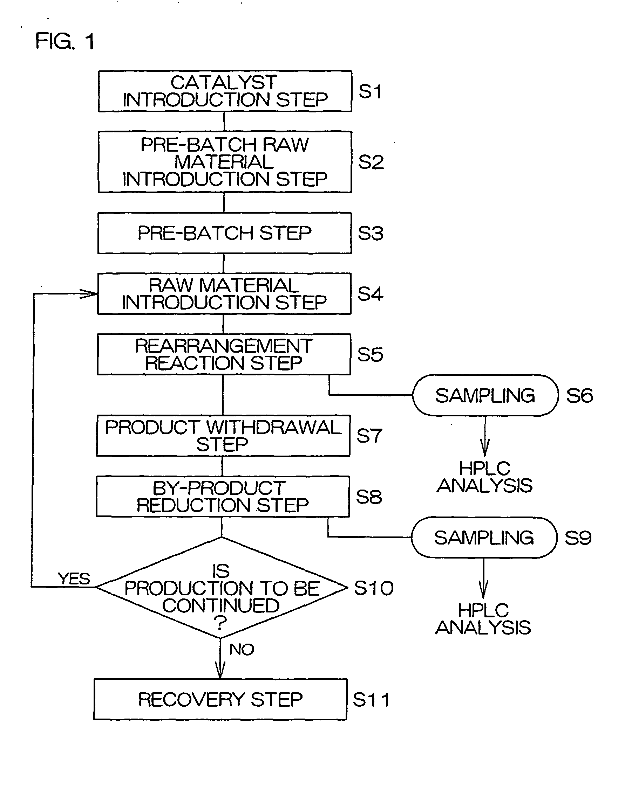

FIG. 1 is a flowchart illustrating one embodiment of a polyamine production process according to the present invention.
Next, an embodiment of the polyamine production process according to the present invention will be described with reference to FIG. 1.
In FIG. 1, in this method, first, an organic sulfonic acid group-containing polysiloxane is introduced into a reaction vessel (S1: catalyst introduction step).

Then, in this method, a solution (hereinafter referred to as reaction solution) containing a condensate of aniline and formaldehyde is introduced into the reaction vessel (S2: pre-batch raw material introduction step).
As a solvent for preparing the reaction solution, for example, a solvent capable of dissolving the condensate of aniline and formaldehyde is used, and more specific examples thereof include aniline; aromatic hydrocarbons such as benzene, toluene and xylene; aliphatic hydrocarbons such as pentane, hexane, heptane, nonane, decane, cyclohexane and decalin; and halogen-substituted hydrocarbons such as dichloromethane, chloroform and carbon tetrachloride. Among them, aniline is preferable. In the case of aniline, excess aniline in the condensation reaction of aniline with formaldehyde can be used intact as a solvent for preparing the reaction solution. Further, when production of polyamine is continuously repeated, such aniline can be used as a raw material of the condensate, which eliminates the need for solvent removal after recovery, thereby achieving reduction of environmental burden.

The amount of the condensate in the reaction solution is, for example, from 30 to 10000 parts by weight, or preferably from 40 to 250 parts by weight, per 100 parts by weight of the reaction solution.
The amount of the reaction solution introduced is set so that the amount of the condensate is, for example, from 100 to 5000 parts by weight, or preferably from 1000 to 5000 parts by weight, per 100 parts by weight of polysiloxane.

Next, in this method, the reaction solution is stirred to disperse the polysiloxane therein to form a dispersed bed (suspended bed) of polysiloxane, while heated whereby the condensate in the reaction solution is subjected to a rearrangement reaction in the presence of polysiloxane (S3: pre-batch step).
In the pre-batch step, heating temperature is set in the range of, for example, 50 to 300°C, preferably 80 to 250°C, or more preferably 80 to 200°C. The heating time is also set in the range of, for example, 0.1 to 40 hours, preferably 1 to 20 hours, or more preferably 1 to 10 hours. Further, the pressure is set in the range of, for example, 0.1 (atmospheric pressure) to 30 MPa. During the heating, if necessary, an inert gas atmosphere can be created with inert gas such as nitrogen and argon. Further, during the heating, if necessary, a solvent to be evaporated can be refluxed.

In this pre-batch step, for example, heating can be performed a plurality of times, or preferably twice. For the heating twice, the second heating temperature can be set to the same or different temperature as/from the first heating temperature, or preferably to a temperature higher than the first heating temperature. When the second heating temperature is set higher than the first one, the second heating temperature is set 10 to 140°C higher, or preferably 10 to 70°C higher, and more specifically to 30°C higher than the first heating temperature. Specifically, the first heating temperature is set in the range of, for example, 50 to 250°C, preferably 80 to 200°C, or more preferably 120 to 170°C, and more specifically to 160°C, while the second heating temperature is set in the range of, for example, 80 to 300°C, preferably 100 to 250°C, or more preferably 180 to 200°C, and more specifically to 190°C.

Further, for the twice heating, the first heating time is set in the range of, for example, 1 to 20 hours, or preferably 3 to 15 hours and more specifically to 6 hours, while the second heating time is set in the range of, for example, 1 to 40 hours, or preferably 6 to 30 hours and more specifically to 15 hours.
The plural times of heating can enhance efficiency of the rearrangement reaction in the pre-batch step.

This pre-batch step allows substantially all of the condensates to be rearranged into the main product (diaminodiphenylmethane and polymethylene polyphenylene polyamine). In such rearrangement, primarily, the bond between methylene group and imino group in N,N'-diphenylmethylenediamine, that is, carbon-to-nitrogen bond, is broken to form a hydrogen-to-nitrogen bond, and is further transferred to the main product.

After completion of the pre-batch step, the reaction solution can be cooled as required to a temperature of, for example, 0 to 55°C, or preferably 10 to 40°C.
Next, in this method, a solution (the same reaction solution as above) containing a condensate of aniline and formaldehyde is poured into the reaction solution after the pre-batch step (S4: raw material introduction step).

The amount of the reaction solution introduced in the raw material introduction step is, for example, from 10 to 500 parts by weight, or preferably 50 to 200 parts by weight, and more specifically 100 parts by weight, per 100 parts by weight of the reaction solution after the pre-batch step.
Then, in this method, the condensate introduced in the reaction solution in the raw material introduction step is subjected to a rearrangement reaction in the presence of polysiloxane (S5: rearrangement reaction step).

For rearrangement reaction of the condensate therein, for example, the reaction solution in which polysiloxane has been dispersed is heated on the same heating condition as that at the pre-batch step (S3). Preferably, the reaction solution is heated on the same heating condition as that in the first of the twice heating at the pre-batch step (S3). When the heating temperature exceeds the above range, a side reaction such as transalkylation occurs in the reaction solution, and heavy compounds are produced. Therefore, the efficiency of the rearrangement reaction decreases, which may cause an increase in time and cost for purifying a product solution described later. On the contrary, when the heating temperature is less than the above range, the efficiency of the rearrangement reaction decreases, which may cause an increase in time and cost for purifying the product solution.

In the rearrangement reaction step, the condensate in the reaction solution contacts polysiloxane, and more specifically, the surface of polysiloxane (primarily non-meso-scale pore). Thus, a product is formed from the condensate, that is, a main product is formed from the condensate and a by-product is also formed (by-produced) therefrom. A solution containing the product formed at the rearrangement reaction step is hereinafter referred to as a product solution.

The efficiency (yield) of the rearrangement reaction of the main product is, for example, from 70 to 100% by mol, or preferably from 80 to 100% by mol. The efficiency of the side reaction of the by-product is, for example, from 0 to 30% by mol, preferably from 0 to 20% by mol.
After completion of the rearrangement reaction step, the product solution can be cooled as required to a temperature of, for example, 0 to 55°C, or preferably 10 to 40°C.

The efficiency of the rearrangement reaction is calculated by collecting a trace amount (e. g. , 100 µL) of the product solution thus cooled as required (S6: sampling), and measuring the collected product solution by liquid chromatography (HPLC method, eluate: mixture of acetonitrile with water, column: ODS column, detecting device: UV detector, wavelength: 200 nm). In the HPLC method, absorption due to the aniline moiety of each compound is detected and a peak appears in the chromatogram. When all of the absorbance values per mole of the main products and the by-products obtained by liquid chromatography at a wavelength of 200 nm are assumed to be the same, the yields of the respective compounds are calculated as the area ratio of the peak area of the main product to the sum total of the peak area of the main product and the peak area of the by-product.

Next, in this method, the product solution after the rearrangement reaction step is partially withdrawn (S7: product withdrawal step (removal step)).
For partial withdrawal of the product solution, for example, stirring is interrupted, the product solution containing polysiloxane is allowed to settle to precipitate the polysiloxane, and the supernatant liquid of the product solution is then removed.

The amount of the product solution withdrawn is from 1 to 90 parts by weight, or preferably from 40 to 80 parts by weight, per 100 parts by weight of the product solution (including the reaction solution in which the condensate has been rearranged at the pre-batch step and the product solution in which the condensate has been rearranged at the rearrangement reaction step).
The product solution thus withdrawn is purified by a known purifying method such as distillation and extraction, to give a main product.

Next, in this method, the peak area of the by-product measured by the HPLC method is reduced (S8: by-product reduction step).
For reduction of the peak area thereof measured by the HPLC method a method of heating a product solution containing the polysiloxane remaining after the product withdrawal step is used.

In the heating method, the product solution containing the polysiloxane remaining after the product withdrawal step is heated on the same heating condition as that at the pre-batch step (S3). Preferably, the product solution is heated on the same heating condition as that in the second of the twice heating at the pre-batch step (S3).
After completion of the by-product reduction step by heating, the product solution can be cooled as required to a temperature of, for example, 0 to 55°C, or preferably 15 to 40°C. The heating method can reduce environmental burden and also reliably reduce the peak area of the by-product measured by the HPLC method.

In the by-product reduction step, when measured by the HPLC method, the peak area of the by-product, relative to the total peak area (100%) of a peak area of the by-product, a peak area of diaminodiphenylmethane, a peak area of polymethylene polyphenylene polyamine, and a peak area of aniline, is reduced to 1.0% or less. In the by-product reduction step, the area ratio of the peak area of the by-product is reduced to preferably 0.85% or less, or more preferably 0.30% or less. When the area ratio exceeds the above range, it is difficult to sufficiently prevent deterioration of catalytic activity and decline in selectivity during the repeated use of polysiloxane.

In other words, when all of the absorbance values per mole of the main products and the by-products obtained by HPLC method at a wavelength of 200 nm are assumed to be the same, the number of moles of the aniline moiety in the by-product is reduced to 1.0% by mole or less of the total number (100%) of moles including the number of moles of aniline moiety in the by-product, the number of moles thereof in diaminodiphenylmethane, the number of moles thereof in polymethylene polyphenylene polyamine and the number of moles of aniline. Further, in other words, on the above assumption, the content ratio of the number of moles of the aniline moiety in the by-product is reduced to 0.85% by mol or less, or preferably to 0.30% by mol or less, in the by-product reduction step.

The peak area of the by-product measured by the HPLC method is calculated by collecting a trace amount (e.g., 100 µL) of the product solution cooled as required (S9: sampling), and measuring the collected product solution by the same HPLC method as above.
Thereafter, in this method, when the production of polyamine is to be continued (S10: YES), each of steps S4 to S9 (reaction and post-treatment) is executed again, and polysiloxane is repeatedly used.

On the contrary, when the production of polyamine is not to be continued (S10: NO), that is, when the production thereof is completed or interrupted, the product solution containing polysiloxane is separated by solid-liquid separation to recover the polysiloxane, and the product solution thus separated by solid-liquid separation is purified by a known purifying method such as distillation and extraction, to give a main product (S11: recovery process).
According to the process for producing a polyamine, even though polysiloxane is repeatedly used, deterioration of catalytic activity and decline in selectivity can be reliably prevented. Therefore, polyamine such as diaminodiphenylmethane and polymethylene polyphenylene polyamine, which are main products, can be produced with a high yield and excellent selectivity over a long period of time.

In the above description, after the catalyst introduction step (S1) in the pre-batch, the pre-batch raw material introduction step (S2) is executed. However, though not shown, for example, the catalyst introduction step (S1) and the pre-batch raw material introduction step (S2) can be simultaneously executed and, in such case, polysiloxane and the reaction solution are simultaneously mixed.
In the above description, the by-product reduction step (S8) is executed after the product withdrawal step (S7). By doing so, when the by-product reduction step is executed by heating, a load on the polysiloxane at the by-product reduction step can be reduced and deterioration of catalytic activity can be suppressed. The execution of the product withdrawal step (S7) can reduce the content (absolute amount) of the by-product, reduce a load on the polysiloxane in the by-product reduction step and suppress deterioration of catalytic activity.

Further, in the above description, the pre-batch raw material introduction step (S2) and the pre-batch step (S3) are executed. However, the catalyst introduction step (S1) and the raw material introduction step (S4) can also be executed in this order without executing the pre-batch raw material introduction step (S2) and the pre-batch step (S3). Preferably, the catalyst introduction step (S1) and the raw material introduction step (S4) are executed in this order without executing the pre-batch raw material introduction step (S2) and the pre-batch step (S3). This process can reduce the number of steps for the production of polyamine, so that polyamine can be conveniently produced.

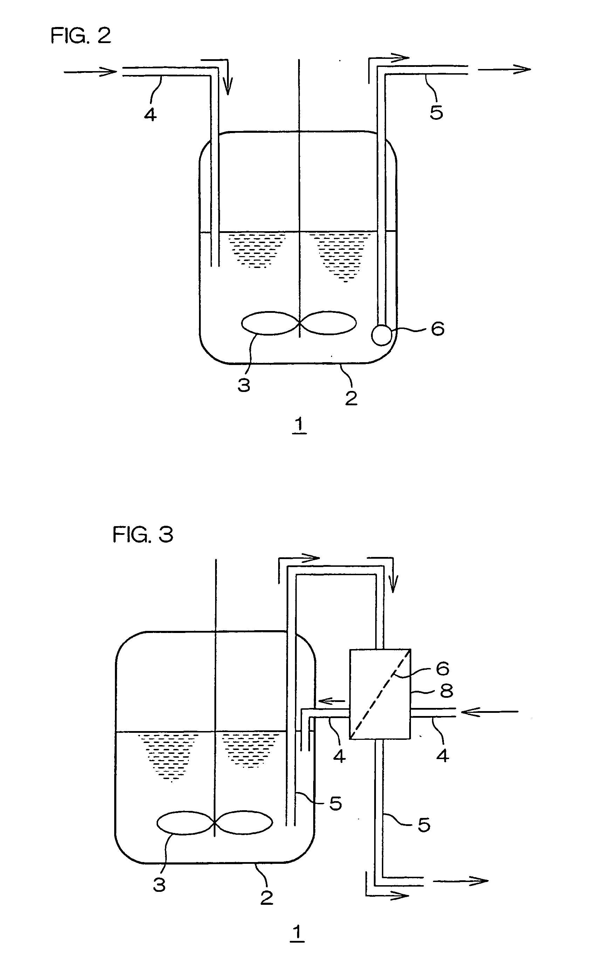

Further, in the above description, all the products (main product and by-product) have been described to be formed from the condensate in the rearrangement reaction step (S5). However, even in a mode in which some of the condensates remain without being subjected to the rearrangement reaction, the remaining condensates can be withdrawn together with the product at the product withdrawal step (S7).
Next, the polyamine production process in which the above-mentioned process is performed using a specific polyamine producing apparatus will be described with reference to FIGS. 2 to 4.

In FIG. 2, the polyamine producing apparatus 1 includes a reaction vessel 2 which is provided with stirring blades 3, a supply line 4 and a discharge line 5.
The reaction vessel 2 is, for example, pressure-resistant and is provided with a jacket, a condenser, a thermometer and a gas introduction line, none of which are shown.
The stirring blades 3 are horizontally rotatably arranged in the lower portion of the reaction vessel 2.

The supply line 4 has an end portion on the downstream side (the downstream side in the liquid flow direction; the same applies to the following.) inserted into the reaction vessel 2, and an end portion on the upstream side (the upstream side in the liquid flow direction; the same applies to the following.) connected to a condensate producing apparatus which produces a condensate.
The discharge line 5 has an upstream side end portion inserted into the reaction vessel 2 and a downstream side end portion connected to a purifying apparatus which purifies a product solution. The upstream side end portion of the discharge line 5 is provided with a filter 6.

Polyamine is produced using the polyamine producing apparatus 1 by the following process: As shown in FIGS. 1 and 2, first, polysiloxane is introduced into the reaction vessel 2 (S1: catalyst introduction step), and then, a reaction solution is fed into the reaction vessel 2 from the supply line 4 (S2: pre-batch raw material introduction step). Then, the reaction solution is heated with the jacket, which is not shown, with stirring with the stirring blades 3, so that a condensate in the reaction solution is subjected to a rearrangement reaction in the presence of polysiloxane (S3: pre-batch step).

Thereafter, the reaction solution is fed into the reaction vessel 2 from the supply line 4 (S4: raw material introduction step), and the condensate in the reaction solution introduced at the raw material introduction step is subjected to a rearrangement reaction in the presence of polysiloxane (S5: rearrangement reaction step). Thereafter, the product solution is sampled (S6: sampling), and the efficiency of the rearrangement reaction is calculated to check the yield of the main product.
Then, the product solution after the rearrangement reaction step is partially removed from the discharge line 5 (S7: product withdrawal step).

Next, a peak area of a by-product measured by the HPLC method is reduced (S8: by-product reduction step). Thereafter, the product solution is collected (S9: sampling), and an area ratio of the peak area of the by-product is calculated to confirm that the area ratio is 1.0% or less.
Thereafter, when the production of polyamine is to be continued (S10: YES), each of steps S4 to S9 (reaction and post-treatment) is executed again, and polysiloxane is repeatedly used.

On the contrary, when the production of polyamine is not to be continued (S10: NO), the product solution containing polysiloxane is recovered from the discharge line 5 from which the filter 6 has been removed. The recovered product solution containing polysiloxane is filtered to recover the polysiloxane, and the product solution thus recovered as filtrate is purified, to give a main product (S11: recovery process).
Next, the polyamine production process in which the above-mentioned process is performed using a polyamine producing apparatus equipped with a filtering device, will be described with reference to FIG. 3. The same reference numerals are used in each of the subsequent figures for the same members as those above, and the description thereof is omitted.

In FIG. 3, the polyamine producing apparatus 1 includes a reaction vessel 2 which is provided with stirring blades 3, a supply line 4, a discharge line 5 and a filtering device 8.
The filtering device 8 is interposed in the supply line 4 and also interposed in the discharge line 5. The filtering device 8 includes a filter 6. The filtering device 8 is also provided with a jacket and a thermometer, which are not shown.

Polyamine is produced using the polyamine producing apparatus 1 by the following process: As shown in FIGS. 1 and 3, first, polysiloxane is introduced into the reaction vessel 2 (S1: catalyst introduction step), and then, a reaction solution is fed into the reaction vessel 2 from the supply line 4 (S2: pre-batch raw material introduction step). Then, the reaction solution is heated with the jacket in the reaction vessel 2 with stirring with the stirring blades 3, so that a condensate in the reaction solution is subjected to a rearrangement reaction in the presence of polysiloxane (S3: pre-batch step).

Thereafter, the reaction solution is fed into the reaction vessel 2 from the supply line 4 (S4: raw material introduction step), and the condensate in the reaction solution introduced at the raw material introduction step is subjected to a rearrangement reaction in the presence of polysiloxane (S5: rearrangement reaction step). Thereafter, the product solution is sampled (S6: sampling), and the efficiency of the rearrangement reaction is calculated to check the yield of the main product.
Then, the product solution after the rearrangement reaction step is partially removed (S7: product withdrawal step). For partial removal of the product solution, first, the discharge line 5 is opened, and at the same time, the supply line 4 is closed. Then, the product solution is withdrawn from the discharge line 5 to such an extent that all of the product solution is substantially removed from the reaction vessel 2. With this withdrawal, most of the product solution is filtered by the filter 6 in the filtering device 8, so that polysiloxane remains on the filter 6 and the remainder of the product solution is left in the filtering device 8.

Next, a peak area of a by-product measured by the HPLC method is reduced (S8: by-product reduction step). In the heating method, for example, the polysiloxane in the filtering device 8 and the remainder of the product solution remaining in the filtering device 8 are heated with the jacket in the filtering device 8. Alternatively, in the method of mixing a large amount of solvent, for example, the large amount of solvent is supplied from the supply line 4 so that the polysiloxane in the filtering device 8 and the remainder of the product solution remaining in the filtering device 8 are flown back into the reaction vessel 2 from the filtering device 8. Then, the product solution is sampled (S9: sampling), and an area ratio of the peak area of the by-product is calculated to confirm that the area ratio is 1.0% or less.

Thereafter, when the production of polyamine is to be continued (S10: YES), each of steps S4 to S9 (reaction and post-treatment) is executed again, and polysiloxane is repeatedly used. In the raw material introduction step (S4) for the second and the subsequent batches, the reaction solution at the raw material introduction step is supplied from the supply line 4 so that the polysiloxane in the filtering device 8 and the remainder of the product solution remaining in the filtering device 8 are flown back into the reaction vessel 2 from the filtering device 8.

On the contrary, when the production of polyamine is not to be continued (S10: NO), inert gas is flown from the upstream side end portion of the discharge line 5 to withdraw the remainder of the product solution remaining in the filtering device 8 from the downstream side end portion of the discharge line 5. The polysiloxane remaining on the filter 6 is recovered during maintenance of the filtering device 8 (S11: recovery process).
When polyamine is produced using the polyamine producing apparatus 1, the jacket in the reaction vessel 2 is used for heating at the rearrangement reaction step (S5) while the jacket in the filtering device 8 is used for heating at the by-product reduction step (S8). Therefore, the temperature for each of the rearrangement reaction step (S5) and the by-product reduction step (S8) can be set to a suitable temperature. As a result, energy efficiency can be improved and production cost can be reduced.

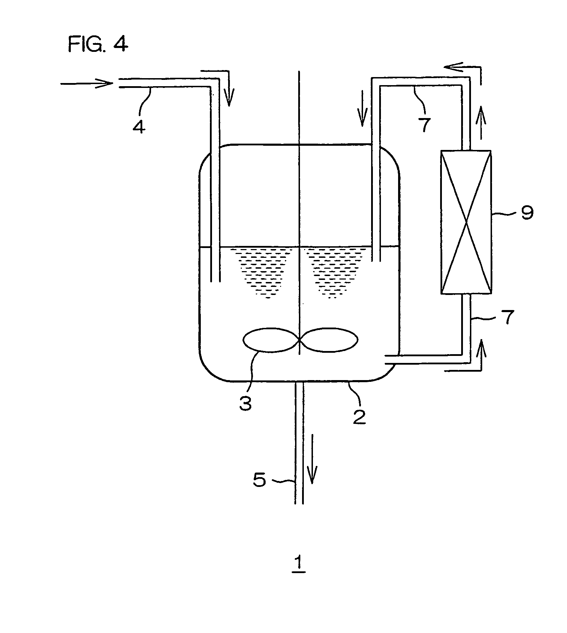

In the above description, the separation device capable of separating polysiloxane in the product solution has been illustrated and described with the filtering device 8. However, it is not limited thereto, and a solid-liquid separator such as a centrifugal separator or a precipitator can also be used.
Next, the polyamine production process in which the above-mentioned process is performed using a polyamine producing apparatus equipped with a catalytic reaction bath, will be described with reference to FIG. 4.

In FIG. 4, the polyamine producing apparatus 1 includes a vessel 2 which is provided with stirring blades 3, a supply line 4, a discharge line 5, a circulation line 7 and a catalytic reaction bath 9.
The vessel 2 is the same as the reaction vessel 2.
The circulation line 7 circulates a reaction solution (or a product solution) of the vessel 2 between the vessel 2 and the catalyst reaction bath 9, and is provided with a circulation pump, which is not shown.

The catalytic reaction bath 9 is interposed in the circulation line 7. The catalytic reaction bath 9 is filled with polysiloxane and is provided with a jacket and a thermometer, which are not shown.
Polyamine is produced using the polyamine producing apparatus 1 by the following process: As shown in FIGS. 1 and 4, in the polyamine producing apparatus 1 provided with the catalytic reaction bath 9 with which polysiloxane has been preliminarily filled (S1: catalyst introduction step), a reaction solution is fed into the reaction vessel 2 from the supply line 4 (S2: pre-batch raw material introduction step). Subsequently, the reaction solution is circulated into the catalytic reaction bath 9 by drive of the circulation pump on the circulation line 7, and also heated with the jacket in the catalytic reaction bath 9, so that the condensate in the reaction solution is subjected to a rearrangement reaction (S3: pre-batch step).

Thereafter, the reaction solution is introduced into the vessel 2 from the supply line 4 (S4: raw material introduction step), and the condensate in the reaction solution introduced at the raw material introduction step is subjected to a rearrangement reaction in the presence of polysiloxane in the catalytic reaction bath 9 (S5: rearrangement reaction step). Thereafter, the product solution is sampled (S6: sampling), and the efficiency of the rearrangement reaction is calculated to check the yield of the main product.
Then, the product solution after the rearrangement reaction step is partially removed from the discharge line 5 (S7: product withdrawal step).

Next, a peak area of a by-product measured by the HPLC method is reduced (S8: by-product reduction step). For reduction of the area ratio of the peak area thereof measured by the HPLC method, for example, the remainder of the product solution remaining in the vessel 2 after the product withdrawal step is circulated between the catalytic reaction bath 9 and the vessel 2 by drive of the circulation pump on the circulation line 7, and also heated with the jacket in the catalytic reaction bath 9, so that the condensate in the product solution is subjected to a rearrangement reaction (S8: by-product reduction step).

Thereafter, the product solution is sampled (S9: sampling), and an area ratio of the peak area of the by-product is calculated to confirm that the area ratio is 1.0% or less.
Thereafter, when the production of polyamine is to be continued (S10: YES), each of steps S4 to S9 (reaction and post-treatment) is executed again, and polysiloxane is repeatedly used.
On the contrary, when the production of polyamine is not to be continued (S10: NO), the product solution is recovered from the discharge line 5, and the polysiloxane is recovered during maintenance of the catalytic reaction bath 9 or the like (S11: recovery process).

When polyamine is produced using the polyamine producing apparatus 1, the jacket in the vessel 2 is used for heating at the rearrangement reaction step (S5) while the jacket in the catalytic reaction bath 9 is used for heating at the by-product reduction step (S8). Therefore, the temperature for each of the rearrangement reaction step (S5) and the by-product reduction step (S8) can be set to a suitable temperature. As a result, energy efficiency can be improved and production cost can be reduced.

In the above description, the catalytic reaction bath 9 is provided as a packed bed of polysiloxane. However, the catalytic reaction bath 9 can also be provided, for example, as a fluid bed of polysiloxane.

### EXAMPLES

While in the following, the present invention is described in further detail with reference to Examples and Comparative Example, the present invention is not limited to any of them by no means.

### (1) Preparation of Organic Sulfonic Acid Group-Containing Polysiloxane

An organic sulfonic acid group-containing polysiloxane was prepared according to Example 1 in Japanese Unexamined Patent Publication No. 2006-83103. The details will be described in the following [1-A] and [1-B].

### [1-A] Preparation of Sulfonic Acid Group-Containing Alkoxysilane

One hundred milliliter of methylene chloride was introduced into a 300-ml two-neck round-bottomed flask equipped with a dropping funnel, 39.1 g (0. 19 mol) of phenyltrichlorosilane was added thereto and the mixture was cooled with ice. Subsequently, 20 ml of a methylene chloride solution with 37.3 g (0.47 mol) of sulfuric anhydride was added dropwise to the methylene chloride solution with phenyltrichlorosilane over 1 hour. After dropwise addition, ambient temperature is set to 60°C, and the methylene chloride solution thus added was subjected to a sulfonation reaction under reflux for 2 hours. Next, ambient temperature is set to 60°C, and 46.0 g of ethanol was added dropwise to the reaction solution over 1 hour with removing hydrogen chloride. Then, ambient temperature is set to 100 °C, and the methylene chloride was distilled off. Then, 46.0 g of ethanol was added dropwise, ambient temperature was set to 100°C, and the added mixture was refluxed to be subjected to an ethoxylation reaction over 2 hours. Thus, 162.7 g of an ethanol solution of sulfonic acid group-containing ethoxy silane containing impurities was obtained.

### [1-B] Preparation of Polysiloxane

The amount 138.0 g of the ethanol solution of sulfonic acid group-containing ethoxy silane obtained in [1-A], 119.0 g (0.57 mol) of tetraethoxysilane and 100 ml of ethanol were introduced into a 1000-ml two-neck round-bottomed flask equipped with stirring blades, and then mixed. The amount 24.0 g of water was added dropwise thereto over 15 minutes and then stirred at 60°C for 3 hours. After the added mixture was allowed to cool, 120.0 g of water was added dropwise thereto for 1 minute and 35 ml of a 28% aqueous ammonia was further added dropwise thereto. Then, the reaction solution was rapidly solidified. The reaction solution thus solidified was allowed to stand for 4 hours, and thereafter, aged at 60°C for 3 days. After aging, the solvent was distilled off at 100°C under a reduced pressure of 1.3 KPa (10 mmHg), and then dried to give a solid. Then, 300 ml of 2N hydrochloric acid was added to the solid and the mixture was stirred for 30 minutes. This operation was repeated twice to convert the sulfonic acid group back to the H+ type. After the hydrochloric acid treatment, the resulting product was washed with 500 ml of ion-exchange water, and then dried at 100°C under a reduced pressure of 1.3 KPa (10 mmHg) for 10 hours. Thus, 55.1 g of polysiloxane having a sulfonic acid group-containing hydrocarbon group was obtained.

When the volume and the specific surface area of the pore of the polysiloxane were measured by a nitrogen adsorption method using a pore size distribution measuring apparatus (ASAP2000, manufactured by Micromeritics Instrument Corporation), the specific surface area was found to be 464 m²/g. The pore having a pore size of 0.9 to 50 nm was found to have a volume of 0.21 cm³/g and the pore having a pore size of 2 to 50 nm was found to have a volume of 0 cm³/g. That is, it was found that meso-scale pores are absent and the surface of the polysiloxane has non-meso-scale pore formed. The content ratio of the sulfonic acid group in the polysiloxane was 1.42 meq/g.

### (2) Preparation of N,N'-diphenylmethylenediamine

Nine hundred grams of aniline (9. 66 mol, guaranteed reagent, manufactured by Kanto Chemical Co, Inc.) was introduced into a 1000-ml round-bottomed flask and then cooled with ice. The amount 161.0 g of formalin (1.98 mol, 37% formaldehyde solution, guaranteed reagent, manufactured by Kanto Chemical Co, Inc.) was added dropwise thereto over 1 hour, and then further stirred for 3.5 hours. The stirred mixture was transferred to a separatory funnel and then allowed to stand for several hours. Thereafter, an aqueous layer was removed therefrom. The mixture was further stirred at 50°C under a reduced pressure of 0.67 KPa (5 mmHg) for 3 hours, and methanol (mixed as a stabilizer in the formalin reagent) contained therein was distilled off.

C¹³-NMR analysis showed that the resulting solution contained 37% by weight of N,N'-diphenylmethylenediamine and 63% by weight of aniline, and when the water content was measured, the solution had a water concentration of 1000 ppm.

### Example 1

### (3) Production of Polyamine

### [3-A] Pre-batch Step

The amount 0.27 g of an organic sulfonic acid group-containing polysiloxane obtained in (1) and 20.0 g of reaction solution of N,N'-diphenylmethylenediamine obtained in (2) were introduced into a 100-ml round-bottomed flask equipped with stirring blades (S1 and S2 with reference to FIG. 1), and the added mixture was heated at a stirring rate of 300 rpm and a temperature of 160 °C for 6 hours under a nitrogen atmosphere, followed by heating at 190°C for 15 hours. Thereafter, the heated mixture was cooled to 40°C, to give a reaction solution containing polyamine (S3).

### [3-B] Rearrangement Reaction Step (In the First Batch)

As a first batch, 20. 0 g of the same reaction solution as above was poured into the reaction solution obtained in the pre-batch step (S4) .

Then, the resulting solution was heated at a stirring rate of 300 rpm and a reaction temperature of 160°C for 6 hours under a nitrogen atmosphere to subject N,N'-diphenylmethylenediamine to rearrangement reaction (S5). Subsequently, the product solution was cooled to 40°C and allowed to stand to precipitate polysiloxane. Thereafter, 20.0 g of supernatant liquid was withdrawn by syringe (S7). Of 20 g of the supernatant liquid thus withdrawn, 100 µL was analyzed by the HPLC method and the area ratio of the peak area of diaminodiphenylmethane to that of polymethylene polyphenylene polyamine was calculated (S6).

The analysis conditions for HPLC are listed below.
HPLC apparatus: LC10A, manufactured by Shimadzu Corporation
Eluate: Mixture of solutions A and B (gradient method)
Solution A (80% by weight of acetonitrile + 20% by weight of water)
Solution B (20% by weight of acetonitrile + 80% by weight of water)
Gradient conditions
At sample injection: Solution A: 99% by weight
30 minutes after sample injection: Solution A: 60% by weight
50 minutes after sample injection: Solution A: 60% by weight
90 minutes after sample injection: Solution A: 0% by weight
100 minutes after sample injection: Solution A: 0% by weight
(Solution B: 100% by weight)
Flow rate: 0.8 ml/min
Column: ODS-3 (ϕ4.6 mm x 250 mm, manufactured by GL Science Inc.)
Column temperature: 40°C
Detecting device: UV detector
Measurement wavelength: 200 nm
Assay: calculated by peak area percentage(%)

### [3-C] By-product Reduction Step (In the First Batch)

Next, the polysiloxane and the product solution, which remain in the reaction vessel, were heated at a stirring rate of 300 rpm and a temperature of 190°C for 15 hours, thereby reducing the peak area of the by-product measured by the HPLC method (S8). Aniline was refluxed by the heating. Thereafter, the product solution was cooled to 40°C and allowed to stand to precipitate polysiloxane. Thereafter, 100.0 µL of supernatant liquid was collected, and the supernatant liquid thus collected was analyzed by the HPLC method and the peak area of aniline, the peak area of the main product, and the peak area of the by-product were calculated (S9). Thus, the area ratio of the peak area of the by-product was calculated.

### [3-D] Rearrangement Reaction Step and By-product Reduction Step (In the Second and the Subsequent Batches)

Then, as the second batch, 20. 0 g of the same reaction solution as above was poured into the polysiloxane and the product solution which remain in the reaction vessel (S4). Steps S4 to S9 were then executed as in the first batch. This process was continued to the 20th batch. The sampling in step S9 was executed only in the first batch, the 6th batch, and from the 10th to the 12th batches.

The area ratios of the peak area of the main product to that of the by-product were listed in the following Table 1.

**[Table 1]**

| Example 1 | | HPLC Method (Measurement Wavelength: 200 nm) | |
|---|---|---|---|
| No. of Batches | Sampling Timing | Peak Area Ratio of Main Product *1 | Peak Area Ratio of Bv-product *2 |
| | | [area %] (VS Peak Area of By-product + Main Product) | [area %] (VS Peak Area of By-product + Main Product + Aniline) |
| 1 | S6 | 87.8 | 3.43 |
| | S9 | 98.7 | 0.41 |
| 2 | S6 | 85.1 | 2.69 |
| 3 | S6 | 86.9 | 2.39 |
| 4 | S6 | 87.6 | 2.08 |
| 5 | S6 | 86.8 | 2.09 |
| 6 | S6 | 86.9 | 2.13 |
| | S9 | 99.5 | 0.15 |
| 7 | S6 | 87.6 | 2.09 |
| 8 | S6 | 87.9 | 2.01 |
| 9 | S6 | 87.1 | 2.00 |
| 10 | S6 | 87.6 | 2.14 |
| | S9 | 99.5 | 0.16 |
| 11 | S6 | 87.8 | 2.16 |
| | S9 | 99.5 | 0.16 |
| 12 | S6 | 85.1 | 2.06 |
| | S9 | 99.5 | 0.18 |
| 13 | S6 | 86.9 | 2.30 |
| 14 | S6 | 87.6 | - |
| 15 | S6 | 86.8 | - |
| 16 | S6 | 86.9 | - |
| 17 | S6 | 87.6 | - |
| 18 | S6 | 87.9 | - |
| 19 | S6 | 87.1 | - |
| 20 | S6 | 87.6 | - |

| | | | |
|---|---|---|---|
| *1: Peak area ratio of main product = (peak area of main product) /(peak area of by-product + peak area of main product) x 100 *2: Peak area ratio of by-product = (peak area of by-product) / (peak area of by-product + peak area of main product + peak area of aniline) x 100 | | | |

As can be seen from Table 1, even if polysiloxane was repeatedly used 19 times, that is, even if the rearrangement reaction step was executed in each of the first to the 19th batches, in production of polyamine in each subsequent batch, that is, in execution of the rearrangement reaction step in each of the second to the 20th batches, the peak area ratio of the main product was as high as 85% or more, so that no deterioration of catalytic activity was found.

### Comparative Example 1

The first to the 5th batches were treated in the same manner as in Example 1 except that the by-product reduction step (S8) by heating and the sampling step (S9) were not executed in each of the batches.
The area ratios of the peak area of the main product and that of the by-product were listed in the following Table 2.

**[Table 2]**

| Comp. Ex. 1 | | HPLC Method (Measurement Wavelength: 200 nm) | |
|---|---|---|---|
| No. of Batches | Sampling Timing | Peak Area Ratio of Main Product *1 | Peak Area Ratio of *2 |
| | | [area %] (VS Peak Area of By-product + Main Product) | [area %] (VS Peak Area of By-product + Main Product + Aniline) |
| 1 | S6 | 81.8 | 2.9 |
| 2 | S6 | 71.8 | 5.3 |
| 3 | S6 | 61.5 | 8.1 |
| 4 | S6 | 41.7 | 13.8 |
| 5 | S6 | 26.4 | 19.1 |

| | | | |
|---|---|---|---|
| *1: Peak area ratio of main product = (peak area of main product) / (peak area of by-product + peak area of main product) x 100 *2: Peak area ratio of by-product = (peak area of by-product) / (peak area of by-product + peak area of main product + peak area of aniline) x 100 | | | |

As can be seen from Table 2, when the rearrangement reaction step was executed in the first to the 4th batches, in production of polyamine in each subsequent batch, that is, in execution of the rearrangement reaction step in each of the second to the 5th batches, the peak area ratio of the main product was as low as 80% or less, and, in addition, the peak area ratio of the main product decreased for each increase in the number of batches, so that catalytic activity was found to be remarkably deteriorated.

### Example 2

The first to the 20th batches were treated in the same manner as in Example 1 except that the sequence of the product withdrawal step (S7) and the by-product reduction step (S8) was reversed, that is, the by-product reduction step (S8) and the product withdrawal step (S7) were executed in this order in Example 1.
More specifically, after the rearrangement reaction step (S5), first, polysiloxane and the product solution were heated, and the peak area of the by-product was then reduced (S8). Thereafter, the product solution was cooled and allowed to stand to precipitate polysiloxane. Thereafter, 100.0 µL of supernatant liquid was collected, and the supernatant liquid thus collected was analyzed by the HPLC method and the peak area of aniline, the peak area of the main product and the peak area of the by-product were calculated (S9). Subsequently, 20.0 g of the supernatant liquid was withdrawn by syringe (S7).

Then, as the second batch, 20.0 g of the same reaction solution as above was poured into the reaction vessel (S4). Steps S4 to S9 were then executed as in the first batch. This process was continued to the 5th batch. The sampling in step S9 was executed only in the first batch.
The area ratios of the peak area of the main product and that of the by-product were listed in the following Table 3.

**[Table 31**

| Example 2 | | HPLC Method (Measurement Wavelength: 200 nm) | |
|---|---|---|---|
| No. of Batches | Sampling Timing | Peak Area Ratio of By-product *2 | Content Ratio of Aniline Moiety of By-product *1 |
| | | [area %] (VS Peak Area of By-product + Main Product) | [area %] (VS Peak Area of By-product + Main Product + Aniline) |
| 1 | S6 | 85.3 | - |
| | S9 | - | 1.0 |
| 2 | S6 | 85.7 | - |
| 3 | S6 | 85.0 | - |
| 4 | S6 | 84.0 | - |
| 5 | S6 | 81.3 | - |

| | | | |
|---|---|---|---|
| *1: Peak area ratio of main product = (peak area of main product) / (peak area of by-product + peak area of main product) x 100 *2: Peak area ratio of by-product = (peak area of by-product) / (peak area of by-product + peak area of main product + peak area of aniline) x 100 | | | |

As can be seen from Table 3, the peak area ratio of the main product after the rearrangement reaction step in each of the second and the third batches was as high as 85% or more, and less repeated use of polysiloxane could prevent deterioration of catalytic activity. However, in the 4th and the 5th batches, the peak area ratio of the main product was less than 85%, and deterioration of catalytic activity was observed by using polysiloxane three or more times (in the 3rd or more batches).

### Example 3

Each batch was treated in the same manner as in Example 1 except that the amount of the supernatant liquid withdrawn in the product withdrawal step (S7) in each of the first to the 20th batches was changed from 20. 0 g to 30. 0 g, and the amount of the reaction solution introduced in the raw material introduction step (S4) in each of the second to the 20th batches was changed from 20.0 g to 30.0 g in Example 1. The sampling in step S9 was executed only in the first to the 13th batches.

The area ratios of the peak area of the main product and that of the by-product were listed in the following Table 4.

**[Table 41**

| Example 3 | | HPLC Method (Measurement Wavelength: 200 nm) | |
|---|---|---|---|
| No. of Batches | Sampling Timing | Peak Area Ratio of Main Product *1 | Peak Area Ratio of By-product *2 |
| | | [area %] (VS Peak Area of By-product + Main Product) | [area %] (VS Peak Area of By-product + Main Product + Aniline) |
| 1 | S6 | 82.0 | 3.62 |
| | S9 | 88.2 | 0.31 |
| 2 | S6 | 85.7 | 2.64 |
| | S9 | 86.7 | 0.21 |
| 3 | S6 | 87.5 | 2.06 |
| | S9 | 86.5 | 0.17 |
| 4 | S6 | 88.1 | 1.89 |
| | S9 | 86.3 | 0.12 |
| 5 | S6 | 88.2 | 1.82 |
| | S9 | 85.8 | 0.11 |
| 6 | S6 | 89.2 | 1.61 |
| | S9 | 85.4 | 1.00 |
| 7 | S6 | 89.4 | 1.54 |
| | S9 | 87.8 | 0.15 |
| 8 | S6 | 88.7 | 1.73 |
| | S9 | 86.7 | 0.14 |
| 9 | S6 | 88.4 | 1.62 |
| | S9 | 86.5 | 0.11 |
| 10 | S6 | 88.9 | 1.54 |
| | S9 | 86.2 | 0.11 |
| 11 | S6 | 87.9 | 1.62 |
| | S9 | 87.2 | 0.12 |
| 12 | S6 | 89.1 | 1.66 |
| | S9 | 87.3 | 0.12 |
| 13 | S6 | 89.3 | 1.66 |
| | S9 | 87.3 | 0.12 |
| 14 | S6 | 89.1 | 1.69 |
| 15 | S6 | 89.1 | 1.68 |
| 16 | S6 | 88.9 | 1.75 |
| 17 | S6 | 88.6 | 1.82 |
| 18 | S6 | 88.5 | 1.86 |
| 19 | S6 | 89.1 | 1.70 |
| 20 | S6 | 88.1 | 1.95 |

| | | | |
|---|---|---|---|
| *1: Peak area ratio of main product = (peak area of main product) / (peak area of by-product + peak area of main product) x 100 *2: Peak area ratio of by-product = (peak area of by-product) / (peak area of by-product + peak area of main product + peak area of aniline) x 100 | | | |

Even if the by-product reduction step (S8) by heating was executed while the weight (10 g) of the remaining product solution in the by-product reduction step (S8) was reduced to half of the weight (20 g) thereof in Example 1, the peak area ratio of the main product was as high as 85% or more, so that no deterioration of catalytic activity of polysiloxane was found.
While the illustrative embodiments of the present invention are provided in the above description, such is for illustrative purpose only and it is not to be construed restrictively. Modification and variation of the present invention that will be obvious to those skilled in the art is to be covered by the following claims.

### INDUSTRIAL APPLICABILITY

The polyamine produced by the polyamine production process of the present invention is used as raw material such as polyurethane.

## Claims

1. A process for producing a polyamine comprising a rearrangement reaction step of:
subjecting a condensate of aniline and formaldehyde to a rearrangement reaction in the presence of an organic sulfonic acid group-containing polysiloxane to form a product containing diaminodiphenylmethane, polymethylene polyphenylene polyamine and a by-product;
a product withdrawal step of partially withdrawing the product solution in an amount 1 to 90 parts by weight per 100 parts by weight of a product solution; and
a reduction step of reducing a peak area of the by-product to 1.0% or less relative to a total peak area of a peak area of the by-product, a peak area of the diaminodiphenylmethane, a peak area of the polymethylene polyphenylene polyamine and a peak area of the aniline when the product is measured by liquid chromatography using a UV detector at a wavelength of 200 nm
wherein the reduction is carried out by heating the product solution containing the polysiloxane remaining after the product withdrawal step.

2. The process for producing a polyamine according to claim 1, wherein the condensate is dissolved in aniline in the rearrangement reaction step.

3. The process for producing a polyamine according to claim 1, wherein
the polysiloxane is formed in the form of a particle having pores, and
a pore having a pore size of 2 to 50 nm is 20% by volume or less per a pore having a pore size of 0.9 to 50 nm.

4. The process for producing a polyamine according to claim 1, wherein
the diaminodiphenylmethane is 4,4'-diaminodiphenylmethane and/or 2,4'-diaminodiphenylmethane;
the polymethylene polyphenylene polyamine is a compound represented by the following formula(s) (1) and/or (2); and
the by-product is at least one selected from N-(4-aminobenzyl)aniline, N-(2-aminobenzyl)aniline, N-methyl-4,4'-methylenedianiline and compounds represented by the following formulas (3) to (5):

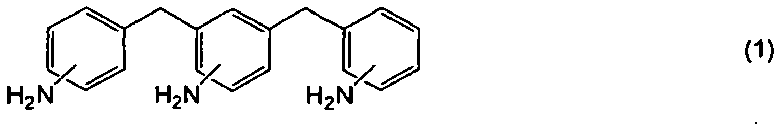

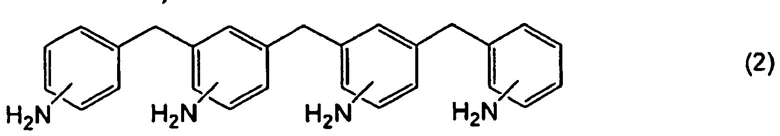

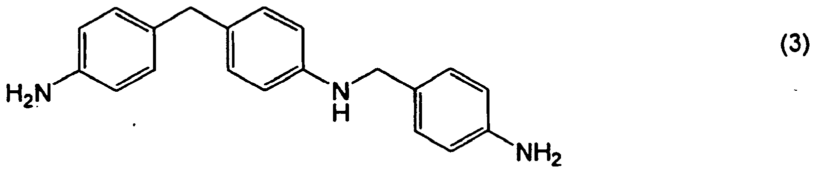

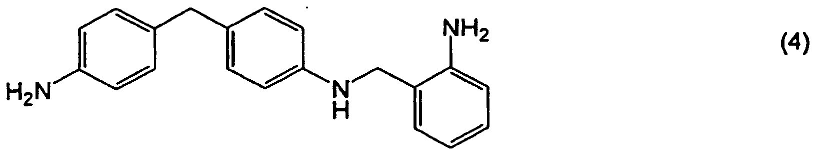

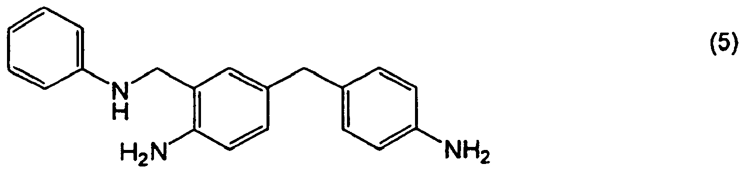

## Patentansprüche

1. Verfahren zur Herstellung eines Polyamins, umfassend einen Umlagerungsreaktionsschritt aus:
Durchführen einer Umlagerungsreaktion mit einem Kondensat aus Anilin und Formaldehyd in Gegenwart eines organische Sulfonsäuregruppen enthaltenden Polysiloxans, um ein Produkt zu bilden, das Diaminodiphenylmethan, Polymethylenpolyphenylenpolyamin und ein Nebenprodukt enthält;
einen Produktentnahmeschritt des teilweisen Entnehmens der Produktlösung in einer Menge von 1 bis 90 Gew.-Teilen pro 100 Gew.-Teilen der Produktlösung; und
einen Reduzierungsschritt des Reduzierens einer Peak-Fläche des Nebenprodukts auf 1,0 % oder weniger in Bezug auf die gesamte Peak-Fläche aus der Peak-Fläche des Nebenprodukts, der Peak-Fläche des Diaminodiphenylmethans, der Peak-Fläche des Polymethylenpolyphenylenpolyamins und der Peak-Fläche des Anilins, wenn das Produkt durch Flüssigchromatografie unter Verwendung eines UV-Detektors bei einer Wellenlänge von 200 nm gemessen wird,
worin die Reduzierung durch Erwärmen der das Polysiloxan enthaltenden Produktlösung, die nach dem Produktentnahmeschritt verbleibt, durchgeführt wird.

2. Verfahren zur Herstellung eines Polyamins gemäß Anspruch 1, worin im Umlagerungsreaktionsschritt das Kondensat in Anilin aufgelöst wird.

3. Verfahren zur Herstellung eines Polyamins gemäß Anspruch 1, worin
das Polysiloxan in Form eines Partikels mit Poren gebildet wird, und
eine Pore mit einer Porengröße von 2 bis 50 nm 20 Vol.% oder weniger in Bezug auf eine Pore mit einer Porengröße von 0,9 bis 50 nm ist.

4. Verfahren zur Herstellung eines Polyamins gemäß Anspruch 1, worin
das Diaminodiphenylmethan 4,4'-Diaminodiphenylmethan und/oder 2,4'-Diaminodiphenylmethan ist;
das Polymethylenpolyphenylenpolyamin eine Verbindung der folgenden Formeln (1) und/oder (2) ist; und
das Nebenprodukt mindestens eines ausgewählt aus N-(4-Aminobenzyl)anilin, N-(2-Aminobenzyl)anilin, N-Methyl-4,4'-methylendianilin und Verbindungen der folgenden Formeln (3) bis (5) ist:

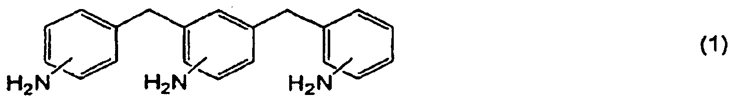

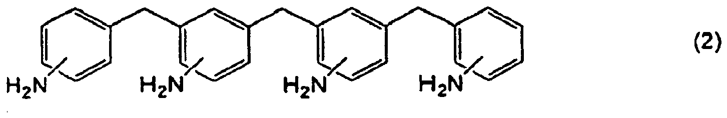

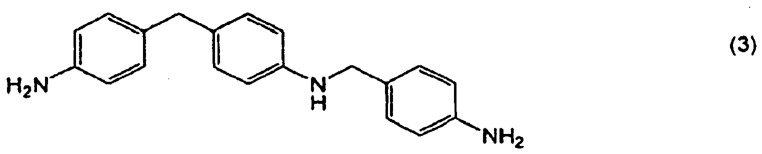

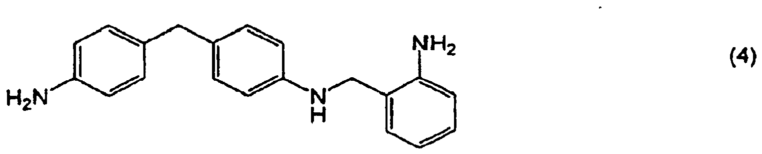

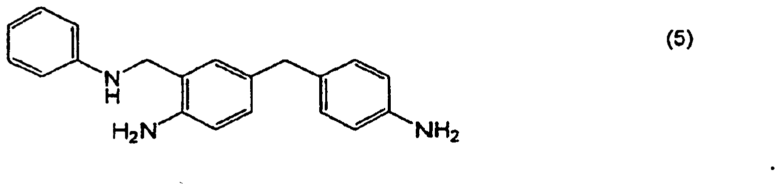

## Revendications

1. Procédé pour produire une polyamine comprenant une étape de réaction de réarrangement de :
soumission d'un condensat d'aniline et de formaldéhyde à une réaction de réarrangement en présence d'un polysiloxane contenant un groupe organique acide sulfonique pour former un produit contenant du diaminodiphénylméthane, une polyméthylène polyphénylène polyamine et un sous-produit ;
une étape d'extraction de produit consistant à extraire partiellement la solution de produit en une quantité de 1 à 90 parties en poids par 100 parties en poids d'une solution de produit ; et
une étape de réduction consistant à réduire une surface du pic du sous-produit à 1,0 % ou moins par rapport à une surface du pic totale d'une surface du pic du sous-produit, une surface du pic du diaminodiphénylméthane, une surface du pic de la polyméthylène polyphénylène polyamine et une surface du pic de l'aniline quand le produit est mesuré par chromatographie liquide en utilisant un détecteur d'UV à une longueur d'onde de 200 nm,
dans lequel la réduction est effectuée en chauffant la solution de produit contenant le polysiloxane restant après l'étape d'extraction de produit.

2. Procédé pour produire une polyamine selon la revendication 1, dans lequel le condensat est dissous dans de l'aniline dans l'étape de réaction de réarrangement.

3. Procédé pour produire une polyamine selon la revendication 1, dans lequel
le polysiloxane est formé sous la forme d'une particule ayant des pores, et
un pore ayant une taille de pore de 2 à 50 nm fait 20 % en volume ou moins pour un pore ayant une taille de pore de 0,9 à 50 nm.

4. Procédé pour produire une polyamine selon la revendication 1, dans lequel
le diaminodiphénylméthane est du 4,4'-diaminodiphénylméthane et/ou du 2,4'-diaminodiphénylméthane ;
la polyméthylène polyphénylène polyamine est un composé représenté par la ou les formule(s) suivante(s) (1) et/ou (2) ; et
le sous-produit est au moins un produit sélectionné à partir de N-(4-aminobenzyl)aniline, N-(2-aminobenzyl)aniline, N-méthyl-4,4 '-méthylènedianiline et des composés représentés par les formules suivantes (3) à (5) :
